(19) Europäisches Patentamt
European Patent Office
Office européen des brevets

(11) **EP 4 304 505 B1**

(12) **FASCICULE DE BREVET EUROPEEN**

(45) Date de publication et mention
de la délivrance du brevet:
**01.07.2026 Bulletin 2026/27**

(21) Numéro de dépôt: **22712975.6**

(22) Date de dépôt: **07.03.2022**

(51) Classification Internationale des Brevets (IPC):
*A61B 17/88* (2006.01)    *A61B 34/10* (2016.01)
*A61B 34/20* (2016.01)

(52) Classification Coopérative des Brevets (CPC):
**A61B 17/8863; A61B 34/10; A61B 34/20;**
A61B 2034/108

(86) Numéro de dépôt international:
**PCT/FR2022/050406**

(87) Numéro de publication internationale:
**WO 2022/189745 (15.09.2022 Gazette 2022/37)**

(54) **DISPOSITIF D'AIDE AU CINTRAGE DE TIGES DE CHIRURGIE**

HILFSVORRICHTUNG ZUM BIEGEN VON CHIRURGISCHEN STANGEN

DEVICE FOR HELPING TO BEND SURGICAL RODS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **08.03.2021 FR 2102186**

(43) Date de publication de la demande:
**17.01.2024 Bulletin 2024/03**

(73) Titulaires:
• **Université de Poitiers**
**86000 Poitiers (FR)**
• **Centre National de la Recherche Scientifique (CNRS)**
**75016 Paris (FR)**
• **Centre Hospitalier Universitaire de Poitiers**
**86000 Poitiers (FR)**

(72) Inventeurs:
• **GERMANEAU, Arnaud**
**86550 Miganloux Beauvoir (FR)**
• **VENDEUVRE, Tanguy**
**86000 Poitiers (FR)**

(74) Mandataire: **Plasseraud IP**
**104 Rue de Richelieu**
**CS92104**
**75080 Paris Cedex 02 (FR)**

(56) Documents cités:
FR-A1- 3 010 628    US-A1- 2009 249 851
US-A1- 2015 320 471    US-A1- 2018 289 396
US-A1- 2019 336 179    US-A1- 2020 015 857

EP 4 304 505 B1

## Description

### Domaine technique

La présente invention relève du domaine de la pose d'implants médicaux et concerne plus particulièrement un dispositif permettant de réaliser un cintrage de tiges utilisées pour la chirurgie rachidienne et vise à assister un chirurgien ou praticien dans le cintrage de tiges implantables en fonction des caractéristiques morphologiques d'un patient.

### Technique antérieure

Pour certaines pathologies, il est connu d'utiliser des implants médicaux lors d'opérations chirurgicales afin de corriger et/ou de stabiliser, de façon temporaire ou permanente, la colonne vertébrale d'un patient. Les implants médicaux peuvent par exemple consister en des tiges fixées à différents points de fixation de la colonne vertébrale par des crochets, des vis ou autres.

Avant d'être implantées dans le patient, les tiges doivent être cintrées, c'est-à-dire déformées dans un ou plusieurs plans pour leur donner une courbure spécifique adaptée à la courbure anatomique du patient.

A l'heure actuelle, le cintrage de tiges implantables est majoritairement réalisé manuellement par le chirurgien au cours de l'opération chirurgicale, ce qui ne permet pas d'obtenir la plupart du temps une correction optimale en utilisant uniquement un examen clinique ou des images médicales du patient et sans disposer de modèles personnalisés.

Il existe également des tiges pré-cintrées éventuellement sur mesure, que le chirurgien n'a pas besoin de cintrer lui-même. Cependant, ces tiges pré-cintrées ne permettent de prendre en compte que certaines courbures anatomiques déterminées à l'avance qui ne sont pas nécessairement complètement adaptée à un patient spécifique.

Pour une tige pré-cintrée sur mesure, en cas de problème de commande ou de livraison ou si elle devient non stérile pendant la chirurgie avant sa mise en place, l'opération peut être compromise.

Afin de pallier ces inconvénients, des solutions existent visant à personnaliser davantage le cintrage de la tige en fonction du patient considéré.

Le document WO2013/191980 A1 décrit un procédé de réalisation d'une tige implantable comportant au préalable la fixation d'attaches dans la colonne vertébrale d'un patient. La courbure de la tige peut être déterminée en utilisant une sonde localisant la position des attaches. Toutefois, cette solution nécessite de déterminer le cintrage de la tige une fois que les attaches dorsales ont déjà été fixées lors de l'opération chirurgicale. Elle est complexe à mettre en œuvre, nécessite de garantir la stérilité de la sonde et rallonge l'intervention.

Le document US 10405935 B2 décrit un dispositif de cintrage d'une tige à partir d'un système de numérisation mesurant la forme de la colonne d'un patient et pilotant une machine de cintrage. Ce dispositif est complexe et coûteux ; de nombreux hôpitaux ne peuvent pas acquérir la machine de cintrage associée.

Il n'y a donc actuellement pas de solution simple, facile à utiliser et adaptée pour la réalisation de tiges ayant un cintrage optimisé et personnalisé.

### Résumé

Au vu de l'art antérieur la présente demande propose en premier lieu un procédé d'aide au cintrage d'une tiges implantables dans un patient selon la revendication 1.

Un tel procédé permet au praticien de réaliser et d'implanter une tige cintrée adaptée au patient lors d'une intervention.

Avantageusement, lesdites mesures de distance et/ou d'angle peuvent comprendre au moins la mesure d'une distance entre les vertèbres L4 et S1 et/ou la distance entre les vertèbres L1 et S1 du patient.

Lesdits paramètres invariants peuvent comporter un paramètre pelvien et une distance entre vertèbres.

Les mesures de distance et/ou d'angle peuvent comprendre une mesure d'incidence pelvienne.

Le procédé peut comporter un classement de la colonne vertébrale selon un type choisi parmi quatre types de colonne vertébrale définissant des modèles de lordose lombaire, cyphose thoracique, lordose cervicale, cyphose cervicale pour affiner la réalisation du cintrage.

Plus particulièrement, un rayon de courbure de cintrage de la tige peut être calculé selon la formule suivante :

[Math 1]

$$s = B(q_i).(1 - A(q_i).x^2)^{\frac{1}{2}}$$

où les coefficients A(q$_i$) et B(q$_i$) sont déterminés à partir des mesures acquises, du type de colonne vertébrale et desdits paramètres invariants, et x est la coordonnée spatiale de la courbure de tige sur le cliché ou la projection.

Le procédé peut comporter le calcul de plusieurs rayons de courbure successifs de la tige.

La fonction $f_n(q_i)$ peut aussi être réalisée à partir d'équations de cercles d'ellipses ou de combinaisons de splines.

La ou les équations peuvent être intégrées en mémoire du dispositif.

Lesdites équations peuvent aussi être adaptées par le chirurgien ou le praticien au moyen d'une formule particulière.

Selon une variante, les paramètres invariants peuvent être identifiés sur le dos du patient ou sur au moins une image du dos du patient dans un plan sagittal.

L'invention propose en outre un dispositif d'aide au cintrage d'une tige implantable dans une colonne vertébrale d'un patient selon la revendication 12, comprenant :

a. des moyens d'acquisition configurés pour acquérir, à partir d'une image du dos du patient, une ou plusieurs mesures de distance et/ou d'angle entre des éléments de la colonne vertébrale du patient ;

b. des moyens de stockage configurer pour stocker une classification de types de colonne vertébrale et des paramètres invariants,

c. des moyens de traitement configurés pour :

i. déterminer un type de la colonne vertébrale du patient selon une classification déterminée;

ii. identifier un ou plusieurs paramètres invariants correspondant au type de colonne vertébrale déterminé;

iii. calculer un ou plusieurs rayons de courbure de cintrage d'une tige en fonction desdites mesures, des paramètres invariants précédemment identifiés, du type de colonne vertébrale déterminé et de paramètres de calcul;

iv. obtenir d'une représentation de la tige cintrée selon lesdits un ou plusieurs rayons de courbure;

d. des moyens d'affichage configurés pour afficher la représentation.

Le dispositif permet ainsi de procéder à la modélisation d'une tige cintrée directement lors d'une intervention à partir de données du patient obtenues lors de l'intervention ou en préopératoire.

Le dispositif peut en outre comprendre une base de données préalablement constituée reliant lesdits types de colonnes vertébrales, liens géométriques, paramètres invariants et comportant des équations adaptées auxdits types de colonnes vertébrales pour calculer ledit modèle du dos du patient et ledit cintrage.

## Brève description des dessins

D'autres caractéristiques, détails et avantages de l'invention apparaîtront à la lecture de la description détaillée ci-après, et à l'analyse des dessins annexés, sur lesquels:

[Fig. 1] montre une vue schématique d'une colonne vertébrale et de ses courbures caractéristiques;

[Fig. 2] montre une classification de différents types de colonnes vertébrales;

[Fig. 3] montre un détail d'une angulation entre des vertèbres d'une colonne vertébrale;

[Fig. 4] est un logigramme exemplifiant l'invention selon un premier mode de réalisation;

[Fig. 5] est un logigramme exemplifiant des étapes de l'invention selon un second mode de réalisation;

[Fig. 6] est une représentation schématique d'un dispositif selon l'invention.

## Description des modes de réalisation

Les dessins et la description ci-après contiennent des éléments non seulement pour servir à mieux faire comprendre la présente invention, mais aussi pour contribuer à sa définition, le cas échéant.

La présente invention vise à assister un chirurgien ou praticien dans le cintrage de tiges en fonction des caractéristiques morphologiques d'un patient. Elle peut en particulier être utilisée pour toute ostéosynthèse de la colonne vertébrale, nécessitant le positionnement et la fixation de vis, de plaques, de clous, de tiges etc. placés en interne, à même l'os, ou en externe à l'aide de fixateurs externes.

Elle peut notamment être utilisé en particulier pour une arthrodèse rachidienne.

Comme décrit par la suite, le chirurgien ou praticien peut grâce à l'invention visualiser et planifier en préopératoire et/ou peropératoire le cintrage de la tige à réaliser pour un patient donné.

Par « préopératoire » et « peropératoire », on comprend respectivement planification avant la chirurgie (patient réveillé) et planification pendant le temps de chirurgie (patient endormi).

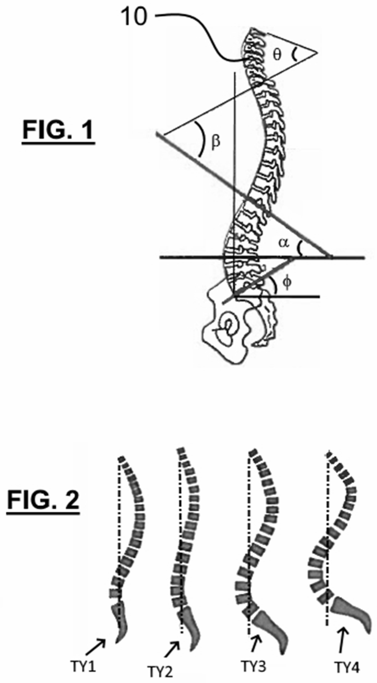

La colonne vertébrale 10 possède généralement plusieurs courbures dans le plan sagittal comme représenté en figure 1. Ces courbures définissent notamment la lordose lombaire $\alpha$, la cyphose thoracique $\beta$ et trois possibilités en cervical $\theta$ (lordose, cyphose et neutre) ainsi que la cyphose sacrée $\phi$.

Il existe ainsi quatre types principaux de colonnes vertébrales, appelés TY1, TY2, TY3, TY4 tels que représentés en figure 2 (Roussouly et al, SPINE, Vol. 30, 3, p. 346-353, 2005). Il est généralement admis que le type TY3 étant la forme habituelle de la colonne vertébrale. Les types TY1, type TY2 et type TY4 sont relatifs à des courbures susceptibles de provenir de certaines pathologies ou de favoriser certaines pathologies. La colonne vertébrale d'un patient donné peut donc être examiné afin d'être catégorisé selon un type particulier.

Pour chacun de ces types de colonne vertébrale, on peut définir des mesures géométriques à partir desquels des paramètres invariants peuvent être identifiés.

Par « mesures géométrique », on comprend une mesure de distance ou d'angle reliant deux ou plus éléments de la colonne vertébrale. Un élément de la colonne vertébrale peut être la position d'une vertèbre, le plan de séparation entre deux vertèbres, ou plus généralement toute position particulière de la colonne vertébrale.

Ainsi, un paramètre invariant identifié peut consister en une mesure de distance ou d'angles, dans un ou plusieurs plans, entre des éléments de la colonne vertébrale. Un paramètre invariant peut notamment être choisi parmi la lordose lombaire, la cyphose thoracique et la cervicale et la cyphose sacrée tels qu'illustrés sur la figure 1. D'autres paramètres invariants sont cependant possibles.

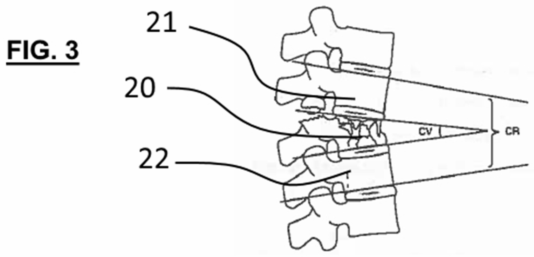

Sur la base du type de colonne vertébrale et des paramètres invariants identifiés, la présente invention permet de définir des angulations optimales que la colonne vertébrale devrait vérifier afin de corriger la pathologie du patient. Dans ce cadre, la figure 3 représente une partie de rachis traumatique au niveau de la vertèbre 20 pour laquelle les angulations CR cyphose régionale entre les vertèbres 21 et 22 entourant la vertèbre 20 et la cyphose vertébrale CV doivent être rétablies pour ramener l'angulation régionale traumatique à une valeur d'angulation physiologique adaptée à la position des vertèbres (par exemple 9° pour T11, 7° pour T12, 1° pour L1, 8° pour L2 ; 18° pour L3, ...).

Par angulation optimale, on entend l'angulation de la colonne vertébrale personnalisée pour un patient donné afin de restituer au mieux sa posture dans une position d'équilibre stable.

La tige cintrée devant être implantée dans le patient peut ainsi être adaptée à ces angulations. Le chirurgien ou praticien peut, avant ou lors de l'opération chirurgicale, cintrer la tige d'une façon personnalisée et spécifique au patient pour restituer au mieux les angulations (en lordose ou en cyphose).

Ainsi, l'invention concerne un dispositif et un logiciel permettant de calculer la ou les courbures (ou rayons de courbure) de la tige nécessaires à partir du modèle de colonne vertébrale et des paramètres invariants d'un patient.

En particulier, le dispositif permet de fournir une représentation de la tige cintrée spécifique au patient. La représentation peut avantageusement être réalisée en temps réel et à une échelle 1 (taille réelle) à l'aide de tout moyen d'affichage.

Par en temps réel, on comprend notamment que l'invention peut être réalisée avant l'opération chirurgicale mais également pendant, de sorte que la tige peut être cintrée pendant le temps opératoire.

La représentation simulée de la tige cintrée à une échelle 1 permet de simplifier la tâche au chirurgien qui peut facilement comparer la tige qu'il a/doit effectivement cintrer avec la représentation obtenue à l'aide du dispositif selon l'invention.

Le chirurgien peut ainsi s'appuyer sur cette représentation pour ensuite réaliser le cintrage de la tige à l'aide d'un dispositif de cintrage dédié, également appelée cintreuse.

Selon une réalisation, le chirurgien dispose alors d'une tige rectiligne et d'une cintreuse, ou « french bender » en anglais (dispositif couramment utilisé au bloc opératoire) mais disposant d'un code couleur selon trois degrés de cintrage fournis par trois galets, ces galets de cintrage étant pourvus de codes couleurs pour définir trois courbures. Le dispositif de l'invention va fournir le code approprié pour que le praticien choisisse la bonne configuration de la cintreuse selon le cas.

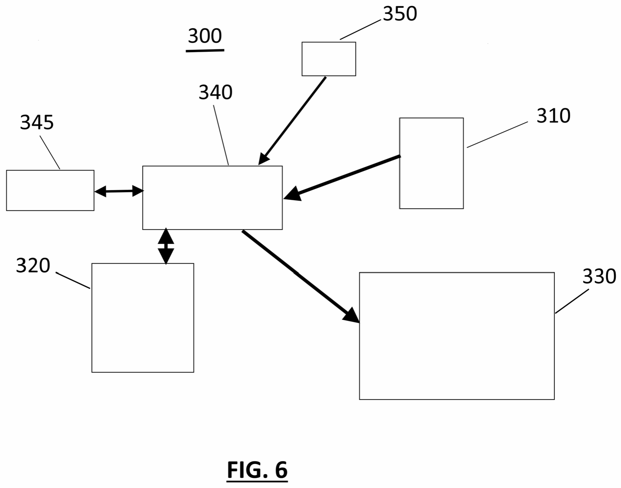

On décrit par la suite le dispositif d'aide au cintrage selon l'invention schématisé en figure 6.

Le dispositif d'aide au cintrage 300 comprend des moyens d'acquisition 310 configurés pour acquérir les mesures de distance et/ou d'angle entre les éléments de la colonne vertébrale.

Les moyens d'acquisition ou interface 310 peuvent par exemple prendre la forme d'une interface homme machine permettant à un chirurgien ou praticien de renseigner les mesures de distance et /ou d'angles lui-même. Selon une autre réalisation, les mesures de distance ou d'angulation sont obtenues directement à partir d'importation d'images médicales, telles que radiographies, IRM, ultrasons ou autres, réalisées à partir d'un appareil d'imagerie médicale intégré au dispositif ou déporté.

Le dispositif comprend en outre des moyens de stockage ou mémoire 320 permettant de stocker les images médicales ou mesures acquises par les moyens d'acquisition du dispositif. La mémoire comprend également une classification de modèle de colonne vertébrale ainsi que les paramètres invariants associés à chaque type de colonne vertébrale.

Le dispositif comporte en outre des moyens de traitement ou calculateur 340, 345, tel qu'un processeur et sa mémoire vive. Comme cela est détaillé par la suite, les moyens de traitement permettent de mettre en œuvre le procédé selon l'invention afin d'obtenir les angulations optimales que la tige, une fois cintrée, devra satisfaire.

Le dispositif comprend un module d'affichage 330, notamment à une échelle 1, de la courbure d'une tige implantable en sorte de permettre à un chirurgien de cintrer ladite tige, par exemple au cours d'une intervention chirurgicale. Ces moyens peuvent comporter pour la visualisation du cintrage des tiges au moins l'un d'une tablette, d'un écran plat, d'un dispositif de vidéo-projection sur une table, un casque de réalité virtuelle ou de tout autre dispositif d'affichage connu.

Par ailleurs, en utilisant lors de l'opération des implants (vis, crochets, clamps) qui, une fois verrouillés, sont perpendiculaires à la tige et parallèles aux plateaux supérieurs des vertèbres, il est ensuite possible de définir l'angulation entre ces implants et d'estimer, en fonction de la distance entre ces implants, le cintrage à réaliser pour ajuster le rayon de courbure à l'angulation. Dans ce cas, l'invention peut être utilisée comme outil de planification peropératoire en mesurant directement la distance entre les vertèbres du patient en cours d'opération. Le dispositif de l'invention permet d'optimiser le cintrage manuel avec les outils habituellement utilisés par les chirurgiens (fers à cintrer, pinces...) et est compatible avec les différents ancillaires utilisés actuellement dans les blocs opératoires.

Le dispositif et le procédé permettent de calculer les courbures pour différents types de tiges, par exemple, dans un cas de traumatisme ou d'un segment dégénératif, le praticien va demander à l'outil de calculer une tige courte reliant 2 à 5 vertèbres. Par contre dans le cas de déformation ou de déséquilibre sagittal, les tiges seront plus longues de 5 à 26 vertèbres et, dans ce cas, plusieurs courbures successives seront calculées avec la méthode de l'invention.

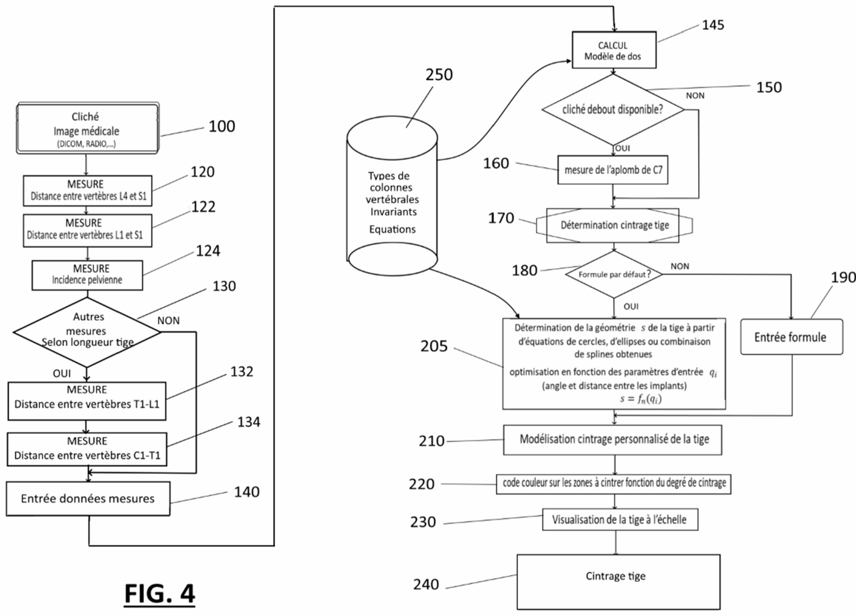

Le logigramme de la figure 4 donne un exemple de mise en œuvre du procédé selon un premier mode de réalisation de l'invention.

Une étape 100, consiste en l'acquisition d'images médicales.

Dans une ou plusieurs étapes suivantes, le praticien effectue des mesures à partir des images médicales précédemment acquises. Selon le premier mode de réalisation, les mesures peuvent être des mesures de distance entre les vertèbres L4 et S1, étape 120, entre les vertèbres L1 et S1, étape 124, et la mesure de l'incidence pelvienne, étape 126. Toutefois, l'invention n'est pas limitée à ces mesures de distance et d'autres mesures, peuvent être effectuées, entre différents éléments de la colonne vertébrale. En particulier, si la tige doit se prolonger vers le haut de la colonne vertébrale, des mesures de distance T1-L1, si la tige se prolonge au niveau thoracique, puis C0-T1, si la tige se prolonge au niveau cervical, peuvent être nécessaires. Dans ce cas, les mesures peuvent être réalisées après un test 130 et peuvent comporter une mesure 132 entre la vertèbre thoracique T1 et la vertèbre lombaire L1 pour une tige se prolongeant au niveau thoracique puis une mesure entre la vertèbre thoracique T1 et la vertèbre cervicale C0 à l'étape 134.

Dans une étape 140, les mesures sont entrées dans le dispositif et, dans une étape 145, le dispositif effectue une classification de la colonne vertébrale. En particulier, le calcul permet de déterminer le type de la colonne vertébrale du patient, par exemple parmi les différents types de colonnes vertébrales TY1, TY2, TY3, TY4 décrits plus haut qui peuvent être regroupés avec d'autres données comme des invariants et des équations utilisables dans une base de données 250.

Après l'étape 140, une ou plusieurs mesures complémentaire peuvent éventuellement être réalisées. Ces mesures complémentaires peuvent notamment comprendre une mesure d'aplomb au niveau de la vertèbre C7 si une image du patient en position debout est disponible (étapes 150, 160).

D'autres informations peuvent également être entrées dans le dispositif, par exemple le type de tige considéré, la présence et la position d'autres implants, etc.

Dans un processus 170, le procédé comporte un algorithme implémentant une formule de calcul de cintrage incluant les paramètres invariants desdits invariants géométriques associés auxdits types de colonnes vertébrales.

Selon l'exemple de la figure 4, le procédé comporte le calcul de détermination du cintrage de la tige. Ce calcul est réalisé ici à l'étape 200 pour laquelle la détermination de la géométrie :

[Math 2]

$$s = f_n(q_i)$$

de la tige est réalisée à partir d'équations de cercles d'ellipses ou de combinaisons de splines et en fonction de paramètres qi dépendant des mesures, du type de colonne vertébrale et d'invariants selon le types de colonne vertébrale.

Les équations peuvent être intégrées en mémoire du dispositif associé ou éventuellement adaptées par le chirurgien ou le praticien au moyen d'une formule particulière 190 selon le choix effectué à l'étape 180.

Ensuite, le dispositif :

    a. - réalise la modélisation 210 à l'échelle 1;

    b. - fournit les codes couleurs 220 pour les différents moyens de cintrages appropriés pour réaliser le cintrage de la tige;

    c. - représente 230 la tige à l'échelle 1 à l'aide des moyens d'affichage.

Le praticien peut alors à l'étape 240 cintrer la tige directement sur le dispositif de cintrage spécifique pourvu des moyens de cintrages appropriés. Ce dispositif peut notamment être équipé de moyens de cintrage comportant des codes couleur selon leur courbures de même couleur que celles issues de la modélisation.

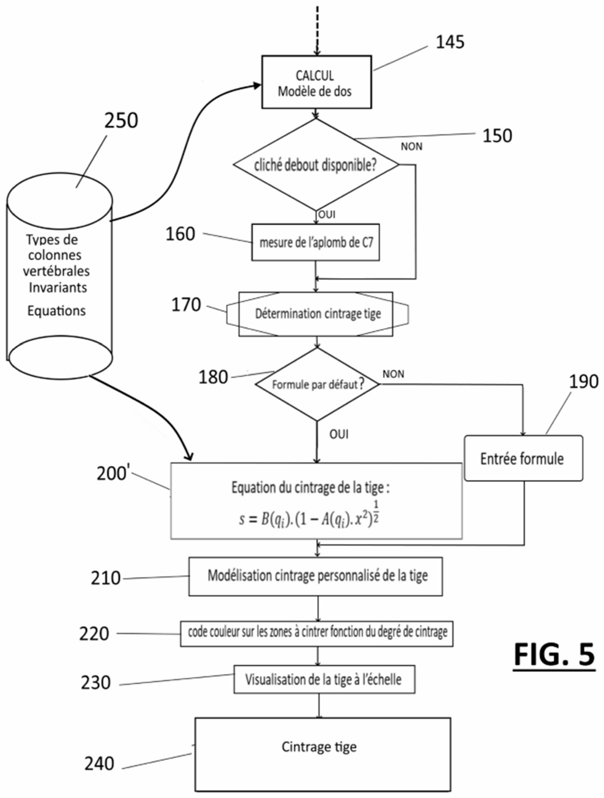

Dans la variante de la figure 5 qui reprend les étapes à partir de l'étape 145, une équation particulière de calcul de cintrage selon laquelle le cintrage s correspond à la formule :

[Math 3]

$$s = B(q_i).\,(1 - A(q_i).\,x^2)^{\frac{1}{2}}$$

Dans cette équation, les coefficients A(qi) et B(qi) sont déterminés à partir des mesures 120, 122, 124 et éventuellement 132, 134 et du type de colonne vertébrale, et x est la coordonnée spatiale de la courbure de tige sur le cliché ou la projection. L'origine des coordonnées est fixée sur la vertèbre S1. Les paramètres qi représentent les données anatomiques d'entrée mesurées par le chirurgien (distances entre les vertèbres, incidence pelvienne).

Lorsque la tige doit avoir plusieurs courbures, l'équation de définition est adaptée pour réaliser les courbures de différents segments de la tige, par exemple segment lombaire, segment thoracique et/ou segment cervical et plusieurs calculs de rayons de courbure successifs de la tige peuvent être réalisées pour la modélisation de la tige.

L'invention ne se limite pas aux exemples décrits ci-avant, seulement à titre d'exemple, mais elle englobe toutes les variantes que pourra envisager l'homme de l'art dans le cadre de la protection définie par les revendications et notamment le dispositif peut comporter d'autres moyens de visualisation à l'échelle 1 que ceux décrits.

**Revendications**

1. Procédé d'aide au cintrage d'une tige implantable dans un patient, le procédé comprenant, à partir d'une acquisition préalable d'une ou plusieurs mesures de distance et/ou d'angle entre des éléments d'une colonne vertébrale du patient réalisées sur une image du dos du patient, les étapes suivantes:

    a) - détermination d'un type de la colonne vertébrale du patient selon une classification ;
    b) - identification d'un ou plusieurs paramètres invariants géométriques correspondant au type de colonne vertébrale déterminé ;
    c) - calcul (200, 200') d'un ou plusieurs rayons de courbure de cintrage d'une tige en fonction desdites mesures, du type de colonne vertébrale déterminé et desdits paramètres invariants précédemment identifiés ;
    d) - obtention d'une représentation de la tige cintrée selon lesdits un ou plusieurs rayons de courbure ; et
    e) - affichage de la représentation à l'échelle 1,
    le procédé comportant un algorithme (200, 200') implémentant une formule de calcul de cintrage incluant les paramètres invariants desdits invariants géométriques associés auxdits types de colonnes vertébrales pour lequel le calcul de détermination d'une géométrie (s) de cintrage de la tige est de la forme

$$s = f_n(q_i)$$

les paramètres qi dépendant des mesures, du type de colonne vertébrale et d'invariants selon le types de colonne vertébrale.

2. Procédé selon la revendication 1, pour lequel lesdites mesures de distance et/ou d'angle comprennent au moins la mesure d'une distance entre les vertèbres L4 et S1 (120) et/ou la distance entre les vertèbres L1 et S1 (122).

3. Procédé selon la revendication 1 ou 2, lequel lesdits paramètres invariants comprennent un paramètre pelvien et une distance entre vertèbres.

4. Procédé selon l'une quelconque des revendications précédentes, pour lequel lesdites mesures de distance et/ou d'angle comprennent une mesure d'incidence pelvienne $\alpha$ (124).

5. Procédé selon l'une quelconque des revendications précédentes pour lequel la colonne vertébrale est classé selon un type choisi parmi quatre types de colonnes vertébrales (TY1, TY2, TY3, TY4) définissant des modèles de lordose lombaire, cyphose thoracique, lordose cervicale, cyphose cervicale et neutre.

6. Procédé selon la revendication 1 pour laquelle la fonction $f_n(q_i)$ est réalisée à partir d'équations de cercles d'ellipses ou de combinaisons de splines.

7. Procédé selon la revendication 6 pour lequel lesdites équations sont intégrées en mémoire du dispositif.

8. Procédé selon la revendication 6 pour lequel lesdites équations sont adaptées par le chirurgien ou le praticien au moyen d'une formule particulière (190).

9. Procédé selon la revendication 1, dans lequel un rayon de courbure de cintrage de la tige est calculé selon la formule suivante:

[Math 4]

$$s = B(q_i).(1 - A(q_i).x^2)^{\frac{1}{2}}$$

où les coefficients A(qi) et B(qi) sont déterminés à partir des mesures acquises, du type de colonne vertébrale et desdits paramètres invariants, et x est la coordonnée spatiale de la courbure de tige sur le cliché ou la projection.

10. Procédé selon l'une quelconque des revendications 1 à 9 comportant le calcul de plusieurs rayons de courbure successifs de la tige.

11. Procédé selon l'une quelconque des revendications précédentes pour lequel lesdits paramètres invariants sont identifiés (120, 122, 124) sur au moins une image (100) du dos du patient dans un plan sagittal.

12. Dispositif d'aide au cintrage d'une tige implantable dans une colonne vertébrale d'un patient comprenant :

a) des moyens d'acquisition (350) configurés pour acquérir à partir d'une image du dos du patient une ou plusieurs mesures de distance et/ou d'angle entre des éléments de la colonne vertébrale du patient;
b) des moyens de stockage (320) configurés pour stocker une classification de types de colonne vertébrale et des paramètres invariants;
c) des moyens de traitement configurés pour :

i) déterminer un type de la colonne vertébrale du patient selon une classification déterminée;
ii) identifier un ou plusieurs paramètres invariants correspondant au type de colonne vertébrale déterminé;
iii) calculer (200, 200') un ou plusieurs rayons de courbure de cintrage d'une tige en fonction desdites mesures, des paramètres invariants précédemment identifiés, du type de colonne vertébrale déterminé et de paramètres de calcul;
iv) obtenir d'une représentation de la tige cintrée selon lesdits un ou plusieurs rayons de courbure ;

d) des moyens d'affichage configurés pour afficher la représentation,
lee moyens de traitement incluant un algorithme (200, 200') implémentant une formule de calcul de cintrage incluant les paramètres invariants desdits invariants géométriques associés auxdits types de colonnes vertébrales pour lequel le calcul de détermination d'une géométrie (s) de cintrage de la tige est de la forme

$$s = f_n(q_i)$$

les paramètres qi dépendant des mesures, du type de colonne vertébrale et d'invariants selon le types de colonne vertébrale.

13. Dispositif selon la revendication 12 comprenant une base de données (250) préalablement constituée reliant lesdits types de colonnes vertébrales, liens géométriques, paramètres invariants et comportant des équations adaptées auxdits types de colonnes vertébrales pour calculer ledit modèle du dos du patient et ledit cintrage.

**Patentansprüche**

1. Verfahren zur Unterstützung beim Biegen einer in einen Patienten implantierbaren Stange, wobei das Verfahren auf der Grundlage einer zuvor erfolgten Erfassung einer oder mehrerer Abstands- und/oder Winkelmessungen zwischen Elementen einer Wirbelsäule des Patienten, die an einem Bild des Rückens des Patienten realisiert wurden, die folgenden Schritte umfasst:

   a) - Bestimmung eines Typs der Wirbelsäule des Patienten gemäß einer Klassifizierung,
   b) - Identifizierung eines oder mehrerer unveränderlicher geometrischer Parameter, die dem bestimmten Wirbelsäulentyp entsprechen,
   c) - Berechnung (200, 200') eines oder mehrerer Krümmungsradien der Biegung einer Stange in Abhängigkeit von den Messungen, dem bestimmten Wirbelsäulentyp und den zuvor identifizierten unveränderlichen Parametern,
   d) - Erhalt einer Darstellung der gebogenen Stange gemäß einem oder mehreren Krümmungsradien; und
   e) - Anzeigen der Darstellung im Maßstab 1:1,

   wobei das Verfahren einen Algorithmus (200, 200') umfasst, der eine Biegeberechnungsformel implementiert, die die unveränderlichen Parameter der geometrischen Invarianten enthält, die den Wirbelsäulentypen zugeordnet sind, für die die Berechnung zur Bestimmung einer Biegegeometrie (s) der Stange die Form

$$s = f_n(q_i)$$

   aufweist, wobei die Parameter qi von den Messungen, dem Wirbelsäulentyp und den Invarianten je nach Wirbelsäulentyp abhängen.

2. Verfahren nach Anspruch 1, wobei die Abstands- und/oder Winkelmessungen wenigstens die Messung eines Abstands zwischen den Wirbeln L4 und S1 (120) und/oder des Abstands zwischen den Wirbeln L1 und S1 (122) umfassen.

3. Verfahren nach Anspruch 1 oder 2, wobei die unveränderlichen Parameter einen Beckenparameter und einen Abstand zwischen Wirbeln umfassen.

4. Verfahren nach einem der vorhergehenden Ansprüche, wobei die Abstands- und/oder Winkelmessungen eine Messung des Beckenwinkels $\alpha$ (124) umfassen.

5. Verfahren nach einem der vorhergehenden Ansprüche, wobei die Wirbelsäule einem Typ zugeordnet wird, der aus vier Wirbelsäulentypen (TY1, TY2, TY3, TY4) gewählt wird, die Modelle für Lendenlordose, Brustkyphose, Halslordose, Halskyphose und Neutralstellung definieren.

6. Verfahren nach Anspruch 1, wobei die Funktion $f_n(q_i)$ anhand von Gleichungen für Kreise, Ellipsen oder Spline-Kombinationen berechnet wird.

7. Verfahren nach Anspruch 6, wobei die Gleichungen im Speicher der Vorrichtung integriert sind.

8. Verfahren nach Anspruch 6, wobei die Gleichungen vom Chirurgen oder Arzt mittels einer speziellen Formel (190) angepasst werden.

9. Verfahren nach Anspruch 1, wobei ein Krümmungsradius der Biegung der Stange anhand der folgenden Formel berechnet wird:

[Math 4]

$$s = B(q_i).(1 - A(q_i).x^2)^{\frac{1}{2}}$$

wobei die Koeffizienten A(qi) und B(qi) anhand der erfassten Messungen, des Wirbelsäulentyps und der unveränderlichen Parameter bestimmt werden und x die räumliche Koordinate der Stangenkrümmung auf dem Bild oder der Projektion ist.

10. Verfahren nach einem der Ansprüche 1 bis 9, das die Berechnung mehrerer aufeinanderfolgender Krümmungsradien der Stange umfasst.

11. Verfahren nach einem der vorhergehenden Ansprüche, wobei die unveränderlichen Parameter auf wenigstens einem Bild (100) des Rückens des Patienten in einer Sagittalebene identifiziert werden (120, 122, 124).

12. Vorrichtung zur Unterstützung des Biegens einer in die Wirbelsäule eines Patienten implantierbaren Stange, umfassend:

    a) Erfassungsmittel (350), die dazu ausgebildet sind, auf der Grundlage eines Bildes des Rückens des Patienten eine oder mehrere Abstands- und/oder Winkelmessungen zwischen Elementen der Wirbelsäule des Patienten zu erfassen,
    b) Speichermittel (320), die dazu ausgebildet sind, eine Klassifizierung von Wirbelsäulentypen und unveränderliche Parameter zu speichern,
    c) Verarbeitungsmittel, die dazu ausgebildet sind:

        i) einen Typ der Wirbelsäule des Patienten nach einer festgelegten Klassifizierung zu bestimmen,
        ii) einen oder mehrere unveränderliche Parameter zu identifizieren, die dem bestimmten Wirbelsäulentyp entsprechen,
        iii) einen oder mehrere Krümmungsradien der Biegung einer Stange in Abhängigkeit von den Messungen, den zuvor identifizierten unveränderlichen Parametern, dem bestimmten Wirbelsäulentyp und Berechnungsparametern zu berechnen (200, 200'),
        iv) eine Darstellung der Stange zu erhalten, die nach dem einen oder den mehreren Krümmungsradien gebogen ist,

    d) Anzeigemittel, die dazu ausgebildet sind, die Darstellung anzuzeigen,

wobei die Verarbeitungsmittel einen Algorithmus (200, 200') umfassen, der eine Biegeberechnungsformel implementiert, die die unveränderlichen Parameter der geometrischen Invarianten umfasst, die den Wirbelsäulentypen zugeordnet sind, für die die Berechnung zur Bestimmung einer Biegegeometrie (s) der Stange die Form

$$s = fn\ (qi)$$

aufweist, wobei die Parameter qi von den Messungen, dem Wirbelsäulentyp und den Invarianten je nach Wirbelsäulentyp abhängen.

13. Vorrichtung nach Anspruch 12, die eine vorab erstellte Datenbank (250) umfasst, die die Wirbelsäulentypen, geometrischen Beziehungen und unveränderlichen Parameter miteinander verknüpft und Gleichungen umfasst, die an die Wirbelsäulentypen angepasst sind, um das Modell des Rückens des Patienten und die Biegung zu berechnen.

**Claims**

1. A method for helping with bending a rod that can be implanted in a patient, the method comprising, on the basis of a prior acquisition of one or more measurements of distance and/or of angle between elements of a spinal column of the patient, these measurements being taken from an image of the back of the patient, the following steps :

   a) - determining a type of spinal column of the patient according to a classification;
   b) - identifying one or more invariable geometrical parameters corresponding to the type of spinal column determined;
   c) - calculating (200, 200') one or more radii of curvature for the bending of a rod as a function of said measurements, of the type of spinal column determined and of said invariable parameters previously identified;
   d) - obtaining a representation of the rod bent to said one or more radii of curvature; and
   e) - displaying the representation at a scale of 1:1,
   the method comprising an algorithm (200, 200') implementing a bending-calculation formula including the invariable parameters of said geometric invariables associated with said types of spinal column, wherein the calculation for determining a geometry (s) for the bending of the rod is of the form:

$$s = f_n(q_i)$$

   the parameters $q_i$ being dependent on the measurements, on the type of spinal column and on invariables according to the types of spinal column.

2. The method as claimed in claim 1, wherein said distance and/or angle measurements comprise at least the measurement of a distance between vertebrae L4 and S1 (120) and/or the distance between vertebrae L1 and S1 (122).

3. The method as claimed in claim 1 or 2, wherein the said invariable parameters comprise a pelvis parameter and an inter-vertebrae distance.

4. The method as claimed in any one of the preceding claims, wherein said distance and/or angle measurements comprise a pelvic tilt measurement $\alpha$ (124).

5. The method as claimed in any one of the preceding claims, wherein the spinal column is classified according to a type selected from four types of spinal column (TY1, TY2, TY3, TY4) defining models of lumbar lordosis, thoracic kyphosis, cervical lordosis, cervical kyphosis and neutral.

6. The method as claimed in claim 1, wherein the function $f_n(q_i$ is produced from equations of circles, of ellipses, or of combinations of splines.

7. The method as claimed in claim 6, wherein said equations are integrated into the memory of the device.

8. The method as claimed in claim 6, wherein said equations are adapted by the surgeon or the practitioner using a specific formula (190).

9. The method as claimed in claim 1, wherein a radius of curvature for the bending of the rod is calculated according to the following formula:

   [Math 4]

$$s = B(q_i).(1 - A(q_i).x^2)^{\frac{1}{2}}$$

   where the coefficients $A(q_i)$ and $B(q_i)$ are determined on the basis of the measurements acquired, of the type of spinal column and of said invariable parameters, and x is the spatial coordinate of the rod curvature in the picture or the projection.

10. The method as claimed in any one of claims 1 to 9 comprising the calculation of several successive radii of curvature of the rod.

11. The method as claimed in any one of the preceding claims, wherein said invariable parameters are identified (120, 122, 124) on at least one image (100) of the back of the patient in a sagittal plane.

12. A device for helping with bending a rod that can be implanted in a spinal column of a patient, comprising:

   a) acquisition means (350) configured to acquire, from an image of the back of the patient, one or more measurements of distance and/or of angle between elements of the spinal column of the patient;
   b) storage means (320) configured to store a classification of types of spinal column and invariable parameters;
   c) processing means configured to:

   i) determine a type of spinal column of the patient according to a determined classification;
   ii) identify one or more invariable parameters corresponding to the type of spinal column determined;
   iii) calculate (200, 200') one or more radii of curvature for the bending of a rod as a function of said measurements, of the invariable parameters previously identified, of the type of spinal column determined and of calculation parameters;
   iv) obtain a representation of the rod bent according to said one or more radii of curvature;

   d) display means configured to display the representation
   the processing means comprising an algorithm (200, 200') implementing a bending-calculation formula including the invariable parameters of said geometric invariables associated with said types of spinal column, wherein the calculation for determining a geometry (s) for the bending of the rod is of the form:

$$s = f_n(q_i)$$

   the parameters $q_i$ being dependent on the measurements, on the type of spinal column and on invariables according to the types of spinal column.

13. The device as claimed in claim 12, comprising a previously-constructed database (250) connecting said types of spinal column, geometric links, invariable parameters and containing equations suited to said types of spinal column for calculating said model of the back of the patient and said bending.

**FIG. 1**

**FIG. 2**

**FIG. 3**

**FIG. 4**

Cliché
Image médicale
(DICOM, RADIO,...) — 100

MESURE
Distance entre vertèbres L4 et S1 — 120

MESURE
Distance entre vertèbres L1 et S1 — 122

MESURE
Incidence pelvienne — 124

Autres mesures
Selon longueur tige — 130
NON

OUI

MESURE
Distance entre vertèbres T1-L1 — 132

MESURE
Distance entre vertèbres C1-T1 — 134

Entrée données mesures — 140

CALCUL
Modèle de dos — 145

cliché debout disponible? — 150
NON

OUI

mesure de l'aplomb de C7 — 160

Détermination cintrage tige — 170

Formule par défaut ? — 180
NON

OUI

Types de colonnes vertébrales Invariants Equations — 250

Détermination de la géométrie $s$ de la tige à partir d'équations de cercles, d'ellipses ou combinaison de splines obtenues

optimisation en fonction des paramètres d'entrée $q_i$ (angle et distance entre les implants)
$s = f_n(q_i)$ — 205

Entrée formule — 190

Modélisation cintrage personnalisé de la tige — 210

code couleur sur les zones à cintrer fonction du degré de cintrage — 220

Visualisation de la tige à l'échelle — 230

Cintrage tige — 240

EP 4 304 505 B1

14

**145** — CALCUL Modèle de dos

**250** — Types de colonnes vertébrales Invariants Equations

**150** — cliché debout disponible? NON

OUI

**160** — mesure de l'aplomb de C7

**170** — Détermination cintrage tige

**180** — Formule par défaut ? NON

**190** — Entrée formule

OUI

**200'** — Equation du cintrage de la tige :
$$s = B(q_i).\left(1 - A(q_i).x^2\right)^{\frac{1}{2}}$$

**210** — Modélisation cintrage personnalisé de la tige

**220** — code couleur sur les zones à cintrer fonction du degré de cintrage

**230** — Visualisation de la tige à l'échelle

**240** — Cintrage tige

**FIG. 5**

**FIG. 6**

# EP 4 304 505 B1

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2013191980 A1

- US 10405935 B2

**Littérature non-brevet citée dans la description**

- **ROUSSOULY et al.** *SPINE,* 2005, vol. 30 (3), 346-353